Europäisches Patentamt

European Patent Office

Office européen des brevets

(11) Publication number: **0 200 464 B1**

(12) **EUROPEAN PATENT SPECIFICATION**

(45) Date of publication of patent specification: **19.08.92**

(51) Int. Cl.5: **C07K 3/02**, A61K 39/00, C12P 21/00, C12N 15/00, G01N 33/577, A61K 49/00, A61K 39/395

(21) Application number: **86303042.5**

(22) Date of filing: **22.04.86**

(54) A tumor associated antigen.

(30) Priority: **22.04.85 US 726202**

(43) Date of publication of application:
**05.11.86 Bulletin 86/45**

(45) Publication of the grant of the patent:
**19.08.92 Bulletin 92/34**

(84) Designated Contracting States:
**AT BE CH DE FR GB IT LI LU NL SE**

(56) References cited:
**EP-A- 0 066 786**

(73) Proprietor: **HYBRITECH INCORPORATED**
**11095 Torreyana Road**
**San Diego California 92126(US)**

(72) Inventor: **Grauer, Lana**
**2470 Malibu Way**
**Del Mar California(US)**
Inventor: **Leung, Julia**
**7924 Camino Jonata**
**San Diego California(US)**
Inventor: **Koda, Joy**
**9993 Avenida Magnifica**
**San Diego California(US)**

(74) Representative: **Goldin, Douglas Michael et al**
**J.A. KEMP & CO. 14, South Square Gray's Inn**
**London WC1R 5EU(GB)**

EP 0 200 464 B1

**Description**

The present invention relates to the characterization of antigens, particularly tumor-associated antigens. In another aspect, it relates to antibodies having specific reactivity with such antigens. In yet another aspect, it relates to methods for producing such antibodies as well as diagnostic and therapeutic uses therefor.

The potential role of monoclonal antibodies in the diagnosis and treatment of cancer has been the focus of much recent investigation and speculation. Of particular interest are their use in immunoassays to monitor the course of disease, e.g., during therapy. Also of particular interest are the potential applications of monoclonal antibodies for tumor imaging and therapy due to their capacity to bind to tumor-associated antigens in vivo.

Developments in monoclonal antibody technology have also made it possible to investigate the antigenic complexity of human tumors. Specifically, the precise immunoreactivity of monoclonal antibodies permits the identification and differentiation of distinct cell-surface antigens expressed by human tumors. The characterization, therefore, of such distinct tumor-associated antigens provides a means to more fully exploit the use of monoclonal antibodies for cancer diagnosis and therapy and to maximize the advances of monoclonal antibody technology.

To date, only a limited number of tumor-associated antigens are well characterized. Additionally, certain tumor-associated antigens useful as diagnostic or prognostic markers may not be present in all patients or during all stages and manifestations of the disease. Accordingly, there exists a need for further identification and characterization of unique tumor-associated antigens.

The present invention is predicated upon the discovery and characterization of a novel tumor associated antigen present in human tissue and cancerous colorectal human cell lines, particularly human colon carcinoma and colon carcinoma cell lines. Accordingly, the invention is directed to a distinct tumor-associated antigen, characterized by its reactivity with monoclonal antibody CCK061.

In accordance with the present invention, antibodies having specificity for the antigen defined herein and methods for the production of such antibodies are provided. Additionally, the invention is directed to the use of such antibodies for the in vitro detection and diagnosis of cancer by immunohistochemical and immunoassay methods. The invention is further directed to the use of such antibodies for the in vivo diagnosis and treatment of cancer in humans.

As indicated above, the present invention provides a novel tumor-associated antigen. In accordance with the invention, monoclonal antibody CCK061, generated by hybrid cell line ATCC Deposit #HB8786, is utilized for the characterization of this previously undescribed antigen.

The physiochemical and immunological properties of the antigen of the present invention, and particularly, its reactivity with monoclonal antibody CCK061, permit its characterization and differentiation from other antigens present in cancerous human cell lines and human tissue, including human tumor tissue. Accordingly, the identifying characteristics and properties of the antigen provided by the present invention are as follows:

a) The antigen is present in human colon carcinoma cell lines. Standard enzyme-linked immunoabsorbent binding assay procedures (ELISA) using monoclonal antibody CCK061 indicate the presence of the antigen in colon carcinoma cell lines but not in such other cell lines as breast carcinoma, prostate carcinoma, bladder carcinoma, adenocarcinoma of lung, and melanoma cell lines.

b) The antigen is produced in the cytosol of normal colon tissue and colon carcinoma. Reactivity of CCK061 with plasma membranes purified from human colon tissue and human colon carcinoma is shown by conventional ELISA techniques. By comparison, CCK061 exhibits no reactivity with membranes purified from other normal or tumor tissues, including breast carcinoma, prostate carcinoma, adenocarcinoma of lung and melanoma.

c) Standard immunohistochemical procedures demonstrate the presence of the antigen in normal colon, colon carcinoma, esophogeal carcinoma and gastric carcinoma as a strong reactivity with CCK061 is shown by immunoperoxidase staining of such tissue. A weak reactivity or no reactivity is shown with breast carcinoma, adenocarcinoma of lung, prostate carcinoma and melanoma.

d) SDS - polyacrylamide gel electrophoresis (SDS-PAGE) and Western immunoblot analysis reveal that the antigen has a molecular weight in the range of about 820,000 to about 960,000. Modification of the antigen by treatment with proteinase K prior to SDS-PAGE and western immunoblot analysis destroys the antigen's reactivity with CCK061, indicating the antigen is a protein.

e) The antigen is glycoprotein in nature. Metabolic labeling of colon carcinoma cells with $C^{14}$-glucosamine and immunoprecipitation of the antigen with CCK061 by standard procedures demonstrate that the antigen contains a carbohydrate component.

f) Isoelectric focusing of the antigen in solution indicates isoelectric points within the ranges of about 4.5

2

to about 5.0 and about 5.1 to about 5.9, and peaks at about 4.9 and about 5.4.

Summarizing the foregoing, the antigen of the present invention, which may be derived from a source selected from human tissue or a cancerous human cell line, is characterized as a glycoprotein having a molecular weight within the range of about 820,000 to about 960,000 and having isoelectric points within the ranges of about 4.5 to about 5.0 and about 5.1 to about 5.9. More specifically, the antigen is characterized as having isoelectric points at about 4.9 and about 5.4. Further, the tumor-associated glycoprotein of the present invention is expressed by normal colon and colon carcinoma and is present in human colon carcinoma cell lines.

Of particular importance in distinguishing the antigen of the present invention from other antigens, including other tumor-associated antigens, is the specificity of monoclonal antibody CCK061 for at least one determinant of the antigen characterized herein. Additionally, the specific reactivity of CCK061 for the antigen defined by the invention provides a means for isolation and purification of the antigen from other material of human origin, and ultimately, characterization of antigenic determinants. Such a purified antigen and determinants thereof are useful in the production of monoclonal and polyclonal antibodies for diagnostic and therapeutic application using techniques well known in the art. For example, murine hybridomas producing monoclonal antibodies may be obtained. In other cases, the antigen may be used to stimulate an immune response in a rabbit, goat or other animal from whose serum polyclonal antibodies may be obtained. In addition, the antigen may be used for the characterization of antibodies of interest, e.g., monoclonal antibodies or antibodies present in human tissue, blood or other body fluids.

In accordance with the present invention, monoclonal antibodies having specificity for the antigen characterized herein, and methods for their production, are provided. Such monoclonal antibodies may be of the IgM or IgG class. Preferably, they are of the IgG class and possess an immunoreactivity with the tumor-associated glycoprotein of the present invention substantially similar to that of monoclonal antibody CCK061. Such monoclonal antibodies are useful in the detection, diagnosis and treatment of cancer in humans, particularly colorectal carcinoma. Further, such antibodies have application in the further isolation and purification of the antigen provided by the invention and the characterization of precise determinants.

Monoclonal antibodies as described above may be produced generally following the method of Kohler and Milstein (Nature 256, 495-497 (1975)). In accordance with the present invention, a mouse or other suitable host is immunized with the purified antigen of the invention or the soluble fraction of human tumor tissue derived from a colon carcinoma. Following immunization, the spleen cells of the immunized mouse are fused with the cells from a suitable mouse myeloma line to obtain a mixture of hybrid cell lines. The hybrid cell lines are cultured in a suitable medium and, thereafter, those hybrid cell lines producing an antibody having specific reactivity with the antigen characterized herein are selected and cloned, and the antibody produced is recovered.

The present invention suggests methods for the in vitro detection and diagnosis of cancer in humans, especially colorectal carcinoma. Characterization of the unique tumor-associated glyprotein of the invention as set forth herein permits its detection in patient tissue samples by immunohistochemical methods and/or in patient fluid samples by in vitro immunoassay methods. A determination of the presence and/or quantity of the tumor-associated antigen of the present invention in patient specimens by immunohistochemical and/or immunoassay procedures is of diagnostic utility and may be indicative of or correlate with the progression of a disease state.

Immunohistochemical methods for the detection of antigens in patient tissue specimens are well known to the art and need not be described in detail. Briefly, in the context of the present invention, a tissue specimen obtained from a suspected cancer patient is contacted with an antibody, preferably a monoclonal antibody, having specificity for the tumor-associated glycoprotein characterized herein. Particularly preferred for use is monoclonal antibody CCK061. The sites at which the antibody is bound is thereafter determined by selective staining of the tissue specimen by standard immunohistochemical procedures.

Similarly, methods for the in vitro detection of antigenic substances in patient fluid samples by immunoassay procedures are well known to the art and require no repetition. For purposes of the present invention, a patient fluid sample is contacted with at least one antibody, preferably a monoclonal antibody, having specific reactivity with the tumor-associated glycoprotein of the invention and the antibody bound to sample components is determined. Qualitative and/or quantitative determinations of the presence of the antigen defined by the invention may be accomplished by competitive or non-competitive immunoassay procedures. Monoclonal antibodies or polyclonal antibodies may be suitably utilized provided such antibodies possess the requisite specificity for the antigen provided by the present invention. Preferably, monoclonal antibody CCK061 is utilized. Additionally, the immunoassays preferred for use are two-site immunometric assays employing monoclonal antibodies which are selected to bind to non-interfering determinants of the antigen characterized herein.

The present invention further suggests methods for the in vivo diagnosis and therapy of cancer in humans, particularly colorectal carcinoma. Methods for tumor localization and detection may be performed, in accordance with the present invention, by administering to a suspected cancer patient a predetermined effective amount of an antibody having specific reactivity with the tumor-associated glycoprotein of the present invention and detecting the sites of localization of the antibody by standard imaging techniques. The antibody, preferably CCK061, is administered to the patient in a pharmaceutically acceptable carrier and labeled, preferably with a radionuclide emitting gamma-radiation, so as to permit detection.

In accordance with methods permitted by the present invention for cancer therapy, a predetermined effective amount of an antibody, preferably a monoclonal antibody, having specificity for the tumor-associated antigen characterized by the invention is administered to a cancer patient. The antibody, preferably CCK061, is administered to the cancer patient in a pharmaceutically acceptable carrier and conjugated with a suitable therapeutic agent, e.g., radioisotopes, preferably emitters of beta particles, drugs, toxins or biological proteins, selected for delivery to the tumor site.

Additionally, in the context of in vivo cancer diagnosis and therapy, those skilled in the art will appreciate that antibody preparations comprising mixtures of antibodies or fragments thereof having specificity for the tumor-associated antigen of the invention may be used in certain instances to enhance the detection, localization and treatment of tumors.

Further in accordance with the present invention, we have unexpectedly discovered that at least one determinant of the antigen characterized herein is expressed by mucin antigens associated with the genetic disease of cystic fibrosis. More specifically, we have found that monoclonal antibody CCK061 is useful for the detection of elevated serum levels of mucin antigens indicative of cystic fibrosis. CCK061 is particularly useful for the detection of cystic fibrosis in patients genetically unable to produce detectable quantities of sialosylated Le$^a$ antigens associated with the disease as CCK061 is reactive with mucin antigens unrelated to the Lewis blood group system. Accordingly, the present invention suggests a method for the detection of cystic fibrosis by contacting a serum sample with monoclonal antibody CCK061 and determining the binding of CCK061 to sample components by means of an immunoassay in which a second antibody, capable of binding with mucin antigens associated with cystic fibrosis, is employed. Preferably, the immunoassay is a two-site immunometric assay and the second antibody is a monoclonal antibody selected such that CCK061 and the second antibody bind to non-interfering determinants of serum mucin antigens associated with cystic fibrosis.

The present invention may be better understood by reference to the following non-limiting example.

EXAMPLE I

Production of Monoclonal Antibody CCK061

To produce hybrid cell line ATCC Deposit #HB 8786 generating monoclonal antibody CCK061, female Balb/c mice (Charles River Breeding Laboratories, Wilmington, MA) were immunized intraperitoneally with 200$\mu$g of tumor cytosol obtained from an autopsy specimen of colon carcinoma 5 times at 14-day intervals. 3 days after the 5th immunization spleens were removed from the mice and a single cell suspension was prepared.

Cell fusion was carried out according to the procedure of Kohler and Milstein, Nature 256, 495-497 (1975). 1 x 10$^8$ splenocytes were fused in 1.0 ml of a fusion medium comprised of 35% polyethylene glycol (PEG 1500) in AP-MEM medium (Flow Laboratories, Inglewood, California) with 2.5 x 10$^7$ P3.653 myeloma cells. Following fusion, cells were cultured in HAT medium (hypoxanthine, aminopterin, thymidine) at 37° in a humidified 5% CO$_2$ incubator.

Antibodies produced by hybridomas were screened by an enzyme-linked immunoabsorbent binding assay (ELISA) on cytosol preparations of the immunizing colon carcinoma vs. normal lung. Antibodies demonstrating a 5-fold or greater reactivity with the tumor cytosol were selected. The monoclonal antibody designated as CCK061 was characterized further and selected for use in the antigen characterization as provided herein.

EXAMPLE II

Antigen Characterization

Monoclonal antibody CCK061 was used to characterize the antigen of the present invention by the procedures set forth herein.

The cell lines utilized for antigen characterization, as well as for screening the reactivity of monoclonal antibody CCK061, were as follows:

| Cell Line | Source |
| --- | --- |
| SW403 AND SW620 Colon Carcinoma | American Type Culture Collection, Rockville, Maryland. |
| T84 Colon Carcinoma | Dr. H. Masui, University of California at San Diego Cancer Center, San Diego, California. |
| SKMel Melanoma | American Type Culture Collection, Rockville, Maryland. |
| Calu-3 Adenocarcinoma of Lung | American Type Culture Collection, Rockville, Maryland. |
| T47D Breast Carcinoma | Dr. Renato Dulbecco, Salk Institute, La Jolla, California. |
| M14 Melanoma | Dr. Ralph Reisfeld, Scripps Clinic and Research Foundation, La Jolla, California. |
| H 907 Bladder Carcinoma | Dr. K.E. Hellstrom, Fred Hutchinson Cancer Research Center, Seattle, Washington. |
| PC-3 Prostate Carcinoma | Dr. Mark Glassey, University of California at San Diego, La Jolla, California. |

Cells were grown in Autopaw media with 8% horse serum and 2% fetal calf serum in a humidified 37° $CO_2$ incubator.

Cell Line ELISA

The presence of antigen reactive with monoclonal antibody CCK061 in human carcinoma cell lines was determined by an enzyme-linked immunoabsorbent binding assay (ELISA) as measured by absorbance at 490 nm.

Cells obtained from the cell lines described above were maintained in tissue culture flasks until approximately 90% confluent. The flasks containing cells and media were frozen at -20°. Flasks were then brought to room temperature and cells removed and counted. Cells were adjusted to $4 \times 10^6$ cells and plated out at $2 \times 10^5$ cells per well on to a Cleveland glassfiber filter microtiter plate. Cells were washed 3 times with 0.3% gelatin, 1% bovine serum albumen in phosphate bufferred saline. Fifty $\mu l$ of monoclonal antibody CCK061 was applied per well and incubated for 1 hour at room temperature. After 5 washes of .3% gelatin/phosphate buffered saline, 50 $\mu l$ of a 1-4000 dilution of peroxidase conjugated goat anti-mouse IgG and IgM was added and incubated for 1 hour. After 6 washes, 200 $\mu l$ of 1 mg/ml o-phenylenediamine, .03% $H_2O_2$ in .1M citrate phosphate buffer was added and incubated with shaking in the dark for 30 minutes. The reaction was quenched with 4N $H_2SO_4$ and read at O.D. 490nm.

As shown by Table I below, CCK061 was reactive with colon carcinoma cell lines SW403 and T84, indicating the presence of the antigen of the invention in human colon carcinoma cell lines. By comparison, CCK061 demonstrated no appreciable reactivity with cell lines such as breast carcinoma, prostate carcinoma, bladder carcinoma, adenocarcinoma of lung and melanoma cell lines.

Membrane/Cytosol ELISA

The presence of antigen reactive with monoclonal antibody CCK061 in purified membrane fractions of normal human tissue and human tumor tissue was determined by an enzyme-linked immunoabsorbent binding assay (ELISA) as measured by absorbance at 490 nm.

Membrane cytosol fractions were prepared from surgical and autopsy specimens of human tissue collected within ten hours after death and stored at -80°C. The tissue was homogenized in 4 volumes of 10mM tris-HCl pH 7.5, 2mM calcium chloride, 2mM phenylmethylsulfonate (homogenization buffer) at 4° in a dounce homogenizer and all subsequent steps were carried out at 4°C. The homogenate was centrifuged at 1000xg for 5 minutes to remove nuclei and intact cells. The supernatant was removed and centrifuged at 130,000 x g for one hour. The supernatant from this high speed centrifugation was removed and designated as the cytosol fraction. The pellet is resuspended in one volume of homogenization buffer and layered on a 40%/20% discontinuous sucrose gradient in 10mM tris-HCl pH 7.2. The gradient was centrifuged at

5

130,000xg for 17 hours. Material at the 40%/20% interphase was pipetted off, diluted 5 fold in homogenization buffer and centrifuged for 60 minutes at 25,000 x g. The pellet was resuspended in homogenization buffer, aliquoted and stored at -80°C.

To perform the membrane/cytosol ELISA, the membrane and cytosol fractions were dried onto 96-well flat bottom polyvinyl-microtiter plates (Dynatech, Alexandria, VA) at 1 $\mu$g and 10 $\mu$g of protein per well, respectively. Plates were washed four times with distilled water and then incubated for 30 minutes at room temperature with 20% horse serum in phosphate-bufferred saline. The buffer was removed and primary antibody applied for one hour. Plates were washed 6 times with tap water prior to the addition of peroxidase conjugated goat anti-mouse IgG and IgM (1:1000 dilution). Plates were incubated for one hour and then washed 5 times with distilled water. Color was developed after the addition of 100 l of 1mg/ml o-phenylenediamine, 0.03% hydrogen peroxide in 0.1M citrate phosphate buffer pH 5.0. Plates were incubated in the dark with shaking for 30 minutes. Wells were quenched by the addition of 50 l per well of $4NH_2SO_4$ and read at 490nm.

As shown by Table II below, CCK061 was reactive with membrane fractions of normal colon and colon carcinoma but unreactive with membranes of other normal tissues and carcinomas, including breast carcinoma, adenocarcinoma of lung, melanoma, and prostate carcinoma. Accordingly, the antigen characterized by CCK061 was demonstrated to be present only in membranes of colon carcinoma and normal colon.

Immunohistochemical Determination

The presence of antigen reactive with CCK061 in normal human tissue and human tumor tissue was determined by immunoperoxidase staining of human tissue.

Sections of frozen tissue blocks, obtained from surgical and autopsy specimens collected within ten hours after death and stored at -80°C were cut 4-6 microns on a microtome/cryostat. The sections, mounted on glass slides, were briefly air-dried and then dipped in acetone. An indirect immunoperoxidase assay, essentially as described by Taylor, Arch.. Pathol. Lab. Med. 102, 113 (1978), was used to stain these slides. Sections were rehydrated by incubating in phosphate-bufferred saline (PBS) for 5 minutes. CCK061 antibody was overlayed onto the the sections and incubated in a humid chamber for one hour. Antibody was washed off the sections with PBS and the sections were then immersed in PBS for 5 minutes. The sections were overlayed with a 1:50 dilution of peroxidase-conjugated goat anti-mouse IgG and IgM antibody; (Tago Chemicals, Burlingame, CA) and incubated for 30 minutes in a humid chamber. Antibody was washed off with PBS. The color reaction was developed with 1mg/ml of diaminobenzidine and 0.03% $H_2O_2$. Slides were counter stained with hematoxylin eosin.

Table III below indicates that CCK061 had a strong reactivity with normal colon mucosa, as measured by the intensity of the staining of tissue, and a weak or no reactivity with other normal tissues. CCK061 demonstrated a weak reactivity with normal breast, lung, and prostate.

Table IV below indicates a strong reactivity of CCK061 with colon carcinoma, esophogeal carcinoma and gastric carcinoma and a weak, or no reactivity, with other carcinomas. A weak reactivity was shown with carcinomas such as breast carcinoma and adenocarcinoma of lung.

Biochemical Analysis of Antigen

The protein nature and molecular weight of the antigen characterized by CCK061 were determined by SDS polyacrylamide gel electrophoresis (SDS-PAGE) and Western immunoblot analysis. 20 $\mu$g of cytosol prepared as described above were dissolved in gel sample buffer (62.5mM tris-HCL pH 6.8, 3% SDS, 10% glycerol, .001% bromophenol blue, 5% mercaptoethanol) and separated by discontinuous SDS-PAGE as described by V. K. Laemmli et al, Nature 227, 680 (1970) on a 3-15% linear gradient gel with a 3% stacking gel. The separated proteins were electrophoretically transferred to nitrocellulose (0.1 m Schleicher and Schuell) at 250mamps overnight according to the method of H. Towbin et al PNAS 76, 4350 (1979). The nitrocellulose replicas were incubated with 3% bovine serum albumin (BSA) in phosphate buffered saline for 30 minutes and then incubated with hybridoma supernatant diluted 1/1000 with 3% BSA-PBS overnight. The nitrocellulose strips were washed with 0.1% tween in PBS for 30 minutes with 5 changes of buffer and incubated with 100,000 cpm/ml 125$_I$-labelled sheep anti-mouse immunoglobulin for 3 hours. After washing with PBS-tween, the nitrocellulose strips were air-dried and exposed on x-ray film (Kodak XAR-5) for 4-72 hours with intensifying screen at -70°C.

To further determine the nature of the antigen, membranes prepared as described above were subjected to the following treatments prior to SDS-PAGE and western immunoblot analysis:

a) Sodium Periodate: 20$\mu$g of cytosol protein was incubated in 50mM sodium periodate for one hour at

4°.

b) Neuraminidase: 20μg of cytosol was adjusted to pH 5-6 with acetic acid and incubated for 1 hour at 37°C with 10mU of clostridium perfringens neuraminidase.

c) Proteinase K: 20 μg of cytosol was incubated in 2.0mg/ml proteinase K for 1 hour at 37°C.

d) Mild Alkali treatment: 20 μg of cytosol was incubated overnight in 0.1N NaOH and then neutralized with .1N HCl.

All reactions were stopped by adding gel sample buffer.

By SDS-PAGE and Western immunoblot analysis the molecular weight of the antigen was determined to be within the range of about 820,000 to about 960,000. Chemical and enzyme modification of the antigen followed by SDS-PAGE and Western immunoblot analysis demonstrated the protein nature of the antigen. Specifically, reactivity of the antigen with CCK061 was shown to be destructible by treatment of the antigen with proteinase K as described above. The loss of reactivity of the antigen with CCK061 did not result from the alternative modifications described above.

Determination of Glycoprotein Nature

The antigen characterized herein was determined to be glycoprotein in nature by immunoprecipitation of $C^{14}$-glucosamine labelled SW403 colon carcinoma cells.

SW403 cells grown in Autopow, 8% horse serum 2% fetal calf serum to confluency in 75 $cm^2$ tissue culture flasks. The media was then supplemented with 50 Ci of $^{14}C$-glucosamine (New England Nuclear, Boston, MA) and incubated for 18 hours at 37° in a humidified incubator supplied with 5% $CO^2$ in air. The cells were washed three times with phosphate bufferred saline (PBS) and then scraped from the flask in 10mls PBS. The cell pellet was washed an additional three times with PBS before being resuspended in 1.0ml lysis buffer (.02M tris-HCl pH 8.0, 1mM EDTA, 0.5% NP-40, 0.5% deoxycholate, 0.5mH phenylmethylsulfonate). After incubation on a rotator at 4° for 1-1/2 hours, the lysate was centrifuged in a microfuge (16,000xg) for 1 minute and the supernatant dialyzed against TBS (.02M tris-HCl pH 8, .15M NaCl, 0.1% sodium azide). The $^{14}C$-labelled lysate was incubated overnight with the CCK061 antibody at 4° on a rotator. Sepharose-bound sheep anti-mouse immunoglobulin was added to the mixture and incubated 3 hours at room temperature. The sepharose-bound material was washed 4 times with .05M tris, .15M NaCl, 0.5% NP-40, .05% deoxycholate, .1% $NaN_3$, 1mg/ml BSA pH 8.1 and another 4 times with the same buffer minus the BSA. Samples were analyzed by SDS-polyacrylamide gel electrophoresis as previously described.

Isoelectric Focusing

Isoelectric focusing of the antigen characterized by CCK061 was determined using an isoelectric focusing column as described.

20 mg of colon carcinoma cytosol prepared as described above were focused in a water-jacketed 110ml isoelectric focusing column in a 0-47% sucrose gradient containing ampholytes of the pH range 3.5-10. The column was prefocused at 600 volts for 12 hours at 4°. The sample was injected into the middle of the gradient and focused an additional 30 hours at 4°. 1 ml fractions were collected from the column and the pH was immediately determined. Fractions were dialyzed against 10mM sodium phosphate pH 7.0 overnight. Aliquots were examined for reactivity with the CCK061 antibody in an ELISA assay on colon carcinoma cytosol.

Isoelectric focusing of the antigen by this technique revealed isoelectric points within the ranges of about 4.5 to about 5.0 and about 5.1 to about 5.9, with peaks of about 4.9 and about 5.4.

TABLE I

| Monoclonal Antibody CCK061 Reactivity With Human Cell Lines | | |
|---|---|---|
| | Cell Line | OD490 |
| SW403 | Colon Carcinoma | .49 |
| T47D | Breast Carcinoma | .03 |
| PC-3 | Prostate Carcinoma | .08 |
| H907 | Bladder Carcinoma | .01 |
| Calu-3 | Adenocarcinoma of Lung | .03 |
| SkMel | Melanoma | .03 |
| M14 | Melanoma | .05 |
| SW620 | Colon Carcinoma | .01 |
| T84 | Colon Carcinoma | .40 |

TABLE II

| Monoclonal Antibody CCK061 Reactivity with Membrane Preparations of Human Tissue | | | |
|---|---|---|---|
| Tumor Membranes | OD490 | Normal Membranes | OD490 |
| Colon Carcinoma | 2.05 | Colon | 2.81 |
| Colon Carcinoma | .78 | Liver | .01 |
| Breast Carcinoma | .00 | Lung | .00 |
| Breast Carcinoma | .03 | Pancreas | .00 |
| Adenocarcinoma of Lung | .03 | spleen | .00 |
| Adenocarcinoma of Lung | .01 | Breast | .00 |
| Squamous Carcinoma of Lung | .01 | Bladder | .00 |
| small Cell Lung Carcinoma | .00 | Brain | .00 |
| Small Cell Lung Carcinoma | .00 | | |
| Melanoma | .01 | | |
| Prostate Carcinoma | .01 | | |
| Prostate Carcinoma | .00 | | |

## TABLE III

### Immunohistological Reactivity of CCK061 on Normal Tissue

| Tissue | No. Positive/Total Examined |
|---|---|
| **Strong Reactivity** | |
| Colon (mucosa only | 4/4 |
| **Weak Reactivity** | |
| Breast | 1/4 |
| Lung | 3/5 |
| Cervix (variable) | 2/2 |
| Esophagus (variable) | 2/2 |
| Prostate | 1/4 |
| Ovary (variable) | 1/2 |
| Thyroid | 1/2 |
| Pancreas | 1/2 |
| **No Reactivity** | |
| Liver | 0/2 |
| Stomach | 0/2 |
| Kidney | 0/2 |
| Bladder | 0/1 |
| Uterus | 0/1 |
| Small Intestine | 0/2 |
| Testis | 0/2 |

TABLE IV
Immunohistological Reactivity of CCK061 on Tumor Tissue

| Tissue | No. Positive/Total Examined |
|---|---|
| **Strong Reactivity** | |
| Colon Carcinoma | 15/18 |
| Esophogeal Carcinoma | 2/2 |
| Gastric Carcinoma | 2/2 |
| | |
| **Weak Reactivity** | |
| Breast Carcinoma (luminal ducts) | 3/7 |
| Adenocarcinoma of Lung | 1/4 |
| Squamous Carcinoma | 2/4 |
| Pancreatic Carcinoma | 2/4 |
| | |
| **No Reactivity** | |
| Small Cell Lung | 0/4 |
| Prostate Carcinoma | 0/4 |
| Hepatoma | 0/2 |
| Melanoma | 0/1 |
| Brain Carcinoma | 0/2 |
| Renal Carcinoma | 0/2 |

The foregoing description of the invention is exemplary for purposes of illustration and explanation. It will be apparent to those skilled in the art that changes and modifications will be possible without departing from the spirit and scope of the invention. It is intended that the following claims be interpreted to embrace all such changes and modifications.

**Claims**

1. A tumor-associate antigen derived from a source selected from human tissue or a cancerous colorectal human cell line, said antigen being characterized as a glycoprotein having a molecular weight within the range of about 820,000 to about 960,000 and isoelectric points within the ranges of about 4.5 to about 5.0 and about 5.1 to about 5.9.

2. The antigen of Claim 1 wherein said isoelectric points are about 4.9 and about 5.4.

3. The antigen of Claim 1 wherein said antigen is further characterized as having at least one antigenic determinant reactive with monoclonal antibody CCK061.

4. The antigen of Claim 3 wherein the reactivity of said antigen with monoclonal antibody CCK061 is destructible by treatment of said antigen with proteinase K.

5. The antigen of Claim 4 wherein said antigen is a tumor-associated antigen expressed by colorectal carcinoma.

6. A method for producing monoclonal antibodies for use in the detection, diagnosis and treatment of cancer in humans comprising:
    immunizing a mouse or other suitable host with purified antigen of Claims 1, 2, 3, 4 or 5 or the soluble fraction of human tumor tissue derived from a human colon carcinoma;
    fusing spleen cells of said immunized mouse or other suitable host with suitable mouse myeloma cells, thereby obtaining a mixture of hybrid cell lines;
    culturing said hybrid cell lines in a suitable melium; selecting and cloning hybrid cell lines producing an antibody having specific reactivity with the antigen of Claims 1, 2, 3, 4 or 5; and

10

recovering monoclonal antibody thus produced.

7. A monoclonal antibody prepared by the method of Claim 6.

8. The monoclonal antibody of Claim 7 wherein said antibody is an IgG antibody.

9. The monoclonal antibody of Claim 7 wherein said antibody is CCK061 generated by hybrid cell line ATCC Deposit #HB8786.

10. A monoclonal antibody of the IgM class having specific reactivity with a tumor-associated glycoprotein characterized as having a molecular weight within the range of about 820,000 to about 960,000, isoelectric points within the ranges of about 4.5 to about 5.0 and about 5.1 to about 5.9, and a reactivity with said monoclonal antibody which is destructible by treatment of said glycoprotein with proteinase K.

11. The monoclonal antibody of Claim 10 wherein said tumor-associated glycoprotein is expressed by colorectal carcinoma.

12. The monoclonal antibody of Claim 11 wherein said antibody is CCK061.

13. A method for the detection and diagnosis of cancer in a suspected cancer patient comprising contacting a tissue specimen obtained from said patient with an antibody having specific reactivity with, the antigen of Claims 1, 2, 3, 4 or 5 and determining the sites on said specimen to which said antibody is bound by immunohistochemical means.

14. The method of Claim 13 wherein said antibody is a monoclonal antibody.

15. The method of Claim 14 wherein said antibody is CCK061.

16. A method for the in vitro detection and diagnosis of cancer in a suspected cancer patient comprising contacting a fluid sample obtained from said patient with at least one antibody having specific reactivity with the antigen or Claims 1, 2, 3, 4 or 5 and determining the binding of said antibody to components of said fluid sample by means of an immunoassay

17. The method of Claim 16 wherein said antibody is a monoclonal antibody.

18. The method of Claim 17 wherein said antibody is CCK061.

19. The method of Claim 16 wherein said immunoassay is a two-site immunometric assay and said antibodies are monoclonal antibodies selected to bind to non-interfering determinants of said antigen.

20. A monoclonal antibody against the antigen of Claims 1, 2, 3, 4 or 5.

21. The monoclonal antibody of Claim 26 wherein said antibody is a murine antibody.

22. Polyclonal antibodies against the antigen of Claims 1, 2, 3, 4 or 5.

23. A method for the in vitro detection of cystic fibrosis comprising contacting a patient serum sample with monoclonal antibody CCK061 and determining the binding of said antibody to components of said sample by means of an immunoassay, said immunoassay employing a second antibody capable of binding at least one serum mucin antigen associated with cystic fibrosis.

24. The method of Claim 29 wherein said immunoassay is a two-site immunometric assay and wherein said second antibody is a monoclonal antibody selected such that said antibody CCK061 and said second antibody bind to non-interfering determinants of said mucin antigen.

**Patentansprüche**

1. Tumorverbundenes Antigen, dessen Herkunft sich von menschlichem Gewebe oder einer cancerösen kolorektalen menschlichen Zellinie ableitet, wobei das Antigen durch ein Glycoprotein charakterisiert ist, das ein Molekulargewicht im Bereich von etwa 820,000 bis etwa 960,000 und isoelektrische Punkte innerhalb der Bereiche von etwa 4.5 bis etwa 5.0 und etwa 5.1 bis etwa 5.9 aufweist.

2. Antigen nach Anspruch 1, wobei die isoelektrischen Punkte bei etwa 4.9 und etwa 5.4 liegen.

3. Antigen nach Anspruch 1, wobei das Antigen weiterhin dadurch charakterisiert ist, daß es mindestens eine mit dem monoklonalen Antikörper CCKO61 reaktive Antigen-Determinante aufweist.

4. Antigen nach Anspruch 3, wobei die Reaktivität des Antigens mit dem monoklonalen Antikörper CCK061 durch Behandlung dem Antigens mit Proteinase K zerstörbar ist.

5. Antigen nach Anspruch 4, wobei das Antigen ein tumorverbundenes Antigen ist, das durch das kolorektale Karzinom exprimiert wird.

6. Verfahren zur Herstellung monoklonaler Antikörper zur Verwendung bei der Erkennung, Diagnose und Behandlung von Krebs bei Menschen, das folgende Schritte einschließt:

   Immunisierung einer Maus oder eines anderen geeigneten Wirts mit gereinigtem Antigen gemäß den Ansprüchen 1, 2, 3, 4 oder 5 oder der löslichen Fraktion eines menschlichen Tumorgewebes, das von einem menschlichen Kolonkarzinom stammt;

   Verschmelzen der Milzzellen der immunisierten Maus oder dem anderen geeigneten Wirts mit geeigneten Mäuse-Myelomzellen, wobei eine Mischung von Hybrid-Zellinien erhalten wird;

   Kultivierung der Hybrid-Zellinien in einem geeigneten Medium;

   Selektieren und Klonen der Hybrid-Zellinien, die einen Antikörper erzeugen, welcher eine spezifische Reaktivität mit dem Antigen gemäß Ansprüchen 1, 2, 3, 4 oder 5 aufweist; und

   Gewinnung des so erzeugten monoklonalen Antikörpers.

7. Monoklonaler Antikörper wie nach dem Verfahren gemäß Anspruch 6 erhalten.

8. Monoklonaler Antikörper gemäß Anspruch 7, wobei es sich bei dem Antikörper um einen IgG-Antikörper handelt.

9. Monoklonaler Antikörper gemäß Anspruch 7, bei dem es sich um den CCK061-Antikörper handelt, der von Hybrid-Zellinien der ATCC-Hinterlegung #HB8786 erzeugt wird.

10. Monoklonaler Antikörper der IgM-Klasse mit einer spezifischen Reaktivität mit einem tumorverbundenen Glycoprotein, das dadurch charakterisiert ist, daß es ein Molekulargewicht innerhalb des Bereichs von etwa 820,000 bis etwa 960,000, isoelektrische Punkte innerhalb der Bereiche von etwa 4.5 bis etwas 5.0 und etwa 5.1 bis etwa 5.9 und eine Reaktivität mit dem monoklonalen Antikörper aufweist, welche durch Behandlung des Glycoproteins mit Proteinase K zerstörbar ist.

11. Monoklonaler Antikörper gemäß Anspruch 10, wobei das tumorverbundene Glycoprotein durch ein kolorektales Karzinom exprimiert wird.

12. Monoklonaler Antikörper gemäß Anspruch 11, wobei es sich um den CCK061-Antikörper handelt.

13. Verfahren zur Erkennung und Diagnose von Krebs bei einem unter Verdacht stehenden Krebspatienten, indem eine von dem Patienten genommene Gewebeprobe mit einem Antikörper in Kontakt gebracht wird, der eine spezifische Reaktivität mit dem Antigen gemäß den Ansprüchen 1, 2, 3, 4 oder 5 aufweist und die Stellen auf der Probe feststellt, an denen der Antikörper durch immunohistochemische Mittel gebunden ist.

14. Verfahren nach Anspruch 13, wobei es sich bei dem Antikörper um einen monoklonalen Antikörper

EP 0 200 464 B1

handelt.

**15.** Verfahren nach Anspruch 14, wobei es sich um den Antikörper CCK061 handelt.

**16.** Verfahren für die in vitro Erkennung und Diagnose von Krebs bei einem unter Verdacht stehenden Krebspatienten, indem eine von dem Patienten genommene flüssige Probe mit wenigstens einem Antikörper in Kontakt gebracht wird, der eine spezifische Reaktivität mit dem Antigen gemäß den Ansprüchen 1, 2, 3, 4 oder 5 aufweist und die Bindung des Antikörpers an die Bestandteile der Flüssigprobe vermittels eines Immunoassays festgestellt wird.

**17.** Verfahren nach Anspruch 16, wobei es sich bei dem Antikörper um einen monoklonalen Antikörper handelt.

**18.** Verfahren nach Anspruch 17, wobei es sich um den Antikörper CCK061 handelt.

**19.** Verfahren nach Anspruch 16, wobei es sich bei dem Immunoassay um einen zwei-Ortimmunometrischen Assay handelt und die Antikörper solche monoklonalen Antikörper darstellen, die an nicht-interferierende Determinanten des Antigens anbinden.

**20.** Monoklonaler Antikörper gegen das Antigen der Ansprüche 1, 2, 3, 4 oder 5.

**21.** Monoklonaler Antikörper gemaß Anspruch 26, wobei es sich bei dem Antikörper um einen Murin-Antikörper handelt.

**22.** Polyklonaler Antikörper gegen das Antigen gemäß den Ansprüchen 1, 2, 3, 4 oder 5.

**23.** Verfahren für die in vitro-Erkennung einer zystischen Fibrose, indem eine Serumprobe eines Patienten mit dem monoklonalen Antikörper CCK061 in Kontakt gebracht und die Bindung des Antikörpers an die Bestandteile der Probe vermittels eines Immunoassays festgestellt wird, wobei bei dem Immunoassay ein zweiter Antikörper eingesetzt wird, der in der Lage ist, mindestens ein mit der zystischen Fibrose verbundenes Serum-Muzinantigen zu binden.

**24.** Verfahren nach Anspruch 23, wobei es sich bei dem Immunoassay um einen Zwei-Ortimmunometrischen Assay handelt und der zweite Antikörper ein solcher monoklonaler Antikörper ist, daß dieser und der Antikörper CCK061 an nicht-interferierende Determinanten des Muzinantigens anbinden.

**Revendications**

**1.** Antigène associé aux tumeurs, dérivé d'une source choisie parmi le tissu humain ou une lignée cellulaire humaine colorectale cancéreuse, ledit antigène étant caractérisé comme une glycoprotéine ayant une masse moléculaire dans la gamme d'environ 820 000 à environ 960 000, et des points isoélectriques dans les gammes d'environ 4,5 à environ 5,0 et d'environ 5,1 à environ 5,9.

**2.** Antigène selon la revendication 1, dans lequel lesdits points isoélectriques sont d'environ 4,9 et d'environ 5,4.

**3.** Antigène selon la revendication 1, dans lequel ledit antigène est caractérisé en outre comme ayant au moins un déterminant antigénique réactif vis-à-vis de l'anticorps monoclonal CCK061.

**4.** Antigène selon la revendication 3, dans lequel la réactivité dudit antigène vis-à-vis de l'anticorps monoclonal CCK061 peut être annihilée par traitement dudit antigène avec la protéinase K.

**5.** Antigène selon la revendication 4, dans lequel ledit antigène est un antigène associé aux tumeurs, exprimé par le carcinome colorectal.

**6.** Procédé de préparation d'anticorps monoclonaux destinés à l'utilisation dans la détection, le diagnostic et le traitement du cancer humain, consistant à :
immuniser une souris, ou un autre hôte approprié, à l'aide de l'antigène purifié selon les

13

revendications 1, 2, 3, 4 ou 5, ou de la fraction soluble de tissu tumoral humain provenant d'un carcinome du colon humain ;

fusionner des cellules de la rate de ladite souris ou de l'autre hôte approprié immunisé, avec des cellules appropriées de myélome de souris, obtenant ainsi un mélange de lignées cellulaires hybrides ;

cultiver lesdites lignées cellulaires hybrides dans un milieu approprié ;

sélectionner et cloner des lignées cellulaires hybrides produisant un anticorps ayant une réactivité spécifique vis-à-vis de l'antigène selon les revendications 1, 2, 3, 4 ou 5 ; et

récupérer l'anticorps monoclonal ainsi obtenu.

7. Anticorps monoclonal préparé par le procédé selon la revendication 6.

8. Anticorps monoclonal selon la revendication 7, dans lequel ledit anticorps est un anticorps IgG.

9. Anticorps monoclonal selon la revendication 7, dans lequel ledit anticorps est le CCK061 généré par la lignée cellulaire hybride ayant le dépôt ATCC # HB8786.

10. Anticorps monoclonal de la classe IgM ayant une réactivité spécifique vis-à-vis d'une glycoprotéine associée aux tumeurs, caractérisé comme ayant une masse moléculaire dans la gamme d'environ 820000 à environ 960 000, des points isoélectriques dans les gammes d'environ 4,5 à environ 5,0 et d'environ 5,1 à environ 5,9, et une réactivité vis-à-vis dudit anticorps monoclonal qui peut être annihilée par traitement de ladite glycoprotéine avec la protéinase K.

11. Anticorps monoclonal selon la revendication 10, dans lequel ladite glycoprotéine associée aux tumeurs est exprimée par le carcinome colorectal.

12. Anticorps monoclonal selon la revendication 11, dans lequel ledit anticorps est le CCK061.

13. Procédé de détection et de diagnostic du cancer chez un patient susceptible d'être atteint d'un cancer, consistant à mettre en contact un spécimen de tissu prélevé chez ledit patient avec un anticorps ayant une réactivité spécifique vis-à-vis de l'antigène selon les revendications 1, 2, 3, 4 ou 5 et à déterminer, par des moyens immunohistochimiques, les sites dudit spécimen auxquels ledit anticorps est lié.

14. Procédé selon la revendication 13, dans lequel ledit anticorps est un anticorps monoclonal.

15. Procédé selon la revendication 14, dans lequel ledit anticorps est le CCK061.

16. Procédé pour la détection in vitro et le diagnostic du cancer chez un patient susceptible d'être atteint d'un cancer, consistant à mettre en contact un échantillon de fluide prélevé chez ledit patient avec au moins un anticorps ayant une réactivité spécifique vis-à-vis de l'antigène selon les revendications 1, 2, 3, 4 ou 5 et à déterminer, au moyen d'un dosage immunologique, la fixation dudit anticorps avec les constituants dudit échantillon de fluide.

17. Procédé selon la revendication 16, dans lequel ledit anticorps est un anticorps monoclonal.

18. Procédé selon la revendication 17, dans lequel ledit anticorps est le CCK061.

19. Procédé selon la revendication 16, dans lequel ledit dosage immunologique est un dosage immunométrique à deux sites et lesdits anticorps sont des anticorps monoclonaux sélectionnés de manière qu'ils se lient à des déterminants indépendants dudit antigène.

20. Anticorps monoclonal dirigé contre l'antigène selon les revendications 1, 2, 3, 4 ou 5.

21. Anticorps monoclonal selon la revendication 20, dans lequel ledit anticorps est un anticorps murin.

22. Anticorps polyclonaux dirigés contre l'antigène selon les revendications 1, 2, 3, 4 ou 5.

23. Procédé pour la détection in vitro de la fibrose cystique, consistant à mettre en contact un échantillon de sérum d'un patient avec un anticorps monoclonal CCK061 et à déterminer la fixation dudit anticorps aux constituants dudit échantillon au moyen d'un dosage immunologique, ledit dosage immunologique utilisant un second anticorps capable de se lier à au moins un antigène de mucine du sérum associé à la fibrose cystique.

24. Procédé selon la revendication 23, dans lequel ledit dosage immunologique est un dosage immunométrique à deux sites et dans lequel ledit second anticorps est un anticorps monoclonal sélectionné de sorte que ledit anticoprs CCK061 et ledit second anticorps se lient à des déterminants indépendants dudit antigène de mucine.